Europäisches Patentamt

European Patent Office

Office européen des brevets

(19)

(11) Veröffentlichungsnummer : **0 054 197**
**B1**

(12)                    EUROPÄISCHE PATENTSCHRIFT

(45) Veröffentlichungstag der Patentschrift :
26.06.85

(51) Int. Cl.⁴ : **A 61 F   2/52**

(21) Anmeldenummer : 81109794.8

(22) Anmeldetag : 20.11.81

(54) **Brustprothese.**

(30) Priorität : 12.12.80 DE 3046784
            12.12.80 DE 3046785
            12.12.80 DE 3046786

(43) Veröffentlichungstag der Anmeldung :
23.06.82 Patentblatt 82/25

(45) Bekanntmachung des Hinweises auf die Patenterteilung : 26.06.85 Patentblatt 85/26

(84) Benannte Vertragsstaaten :
AT BE CH DE FR GB IT LI NL SE

(56) Entgegenhaltungen :
DE-A- 2 742 394
US-A- 3 896 506
US-A- 3 911 503

(73) Patentinhaber : **Beiersdorf Aktiengesellschaft
Unnastrasse 48
D-2000 Hamburg 20 (DE)**

(72) Erfinder : **Prahl, Jan
Nutzfelder Weg 13
D-2127 Rullstorf (DE)**

## Beschreibung

Die Erfindung betrifft eine Brustprothese aus einem elastischen Kunststoff, insbesondere aus Silikonkautschuk-Masse, mit einem der weiblichen Brust entsprechenden Brustformteil mit einer der menschlichen Körperform angepaßten Anlagefläche, wobei der Brustformteil körperauflageflächenseitig eine Ausnehmung und einen in dieser gehaltenen adapterartigen Formteil aufweist, dessen außenseitige, dem Körper zugewandte Wandfläche mit einer Beschichtung aus einer hautverträglichen Haftklebemasse versehen ist.

Der Versorgungskreis brustamputierter Frauen zeigt eine zunehmende Tendenz, wobei die Amputation meist die Folge von bösartigen Tumoren ist. Je nach Größe der Tumorausweitung entstehen bei der Amputation unterschiedlich große Narben und oftmals schlecht gedeckte Körperflächen. Die dann nach den Amputationen entstehenden Narben weisen eine Überempfindlichkeit gegen Druckkanten und Scheuerstellen auf. Hinzu kommt noch, daß durch die Brustamputation die symmetrische Gewichtsverteilung auf die Wirbelsäule gestört ist, so daß an eine Brustprothese folgende Grundforderungen gestellt werden : Die Prothese muß der Körperform weitgehend angepaßt sein und an der Körperauflageseite eine weitgehend geschlossene Anlagefläche bilden, die Gewichtsbestimmung der Brustprothese muß so gewählt sein, daß sie in der Gewichtsmasse der Gegenseite der meist voll erhaltenen Brust entspricht, wobei vor allem Veränderungen des Schultergürtels und der Wirbelsäule vermieden werden sollen, das Volumen der Brustprothese soll dabei so aufgebaut sein, daß diese in ihrem Schwingungsverhalten der natürlichen Brust sehr nahe kommt und darüber hinaus soll die Oberfläche derartiger Brustprothesen aus physiologisch unbedenklichem Material bestehen, da oftmals Narben mit offenen Stellen anzutreffen sind.

Es sind bereits eine ganze Reihe von Brustprothesen in den verschiedensten Ausführungsformen bekannt, die diesen Anforderungen versuchen gerecht zu werden. Beispielsweise bekannt ist eine Brustprothese, bestehend aus einem biegsamen, einteiligen, luftfreien, der Brustform nachgebildeten, schalenförmigen Körper (DE-U-7 603 424). Bei dieser Brustprothese besteht der Körper aus additionsvernetzendem Zweikomponenten-Silikonkautschuk, dessen Ober- und Unterseiten von je einer Kunststoff-Folie abgedeckt sind, die längs des Randes des schalenförmigen Körpers miteinander verschweißt sind. Derartige, mit einem Hohlraum im Anlagenseitenbereich versehene Brustprothesen werden in Schalenform gearbeitet, jedoch erfüllen sie nicht die wesentlichen Forderungen, die an eine Brustprothese gestellt werden. So sind vor allen Dingen die Probleme der Anlagefläche ungenügend gelöst und die vertikalen Schwingungsausgleiche, wie diese vor allem für größere Brustformen von Bedeutung sind, werden nicht erreicht.

Darüber hinaus ist eine Brustprothese für Frauen mit einem die eigentliche Mamma nachbildenden Hauptteil und einem sich hieran nach einer Seite hin anschließenden Hilfsteil zur Schaffung eines kontinuierlichen Überganges zwischen der Prothese und dem Körper der Trägerin in der Nähe der Achselhöhle, wobei der Hauptteil und der Hilfsteil von einem von einer Hülle umschlossenen elastischen Medium gebildet werden, bekannt geworden, bei der das elastische Medium aus einem additionsvernetzendem Silikonkautschuk und die Hülle von einer thermoplastisch linear vernetzten, reißfesten Polyurethanfolie gebildet sind (DE-U-7 602 166). Mit einer derart ausgebildeten Brustprothese soll die sonst bei mit Flüssigkeit gefüllten Brustprothesen auftretende Gefahr eines schnellen Ausströmens des elastischen Formgebungsmediums bei einer Beschädigung des Brustprothesenkörpers dadurch vermindert werden, daß anstelle der Flüssigkeit eine Silikonfüllung gewählt wird.

Diese und andere bekannte Brustprothesen erfüllen angenähert die an Brustprothesen gestellten Grundforderungen, jedoch sind sie in Bezug auf ihre konstruktiven Ausgestaltungen und verwendeten Werkstoffe nicht so ausgebildet, daß bei einem Berührungskontakt keine Unterschiede mehr zur natürlichen Brust spürbar sind. Darüber hinaus vermitteln sie zwar ihrer Trägerin während des Tragens der Brustprothese innerhalb eines Büstenhalters weitgehend das Gefühl des Vorhandenseins einer natürlichen Brust. Besonders nachteilig wird es jedoch seitens der Trägerin empfunden, wenn der Büstenhalter abgelegt und somit auch gleichzeitig die Brustprothese mit entfernt werden muß.

Um diesen Nachteil zu beseitigen ist eine der natürlichen weiblichen Brust möglichst naturgetreu nachgebildete Brustprothese aus Kunststoffmaterial bekannt geworden (DE-A-2 742 394), die die im Oberbegriff des Anspruchs 1 genannten Merkmale aufweist und die mit mehreren einzelnen Haftbefestigern oder Haftringen versehen ist, die an bzw. unter dem Rand angebracht sind und mittels derer die Brustprothese an die Haut des menschlichen Körpers angeheftet werden kann, so daß eine derartige Brustprothese über längere Zeiträume mit dem Körper verbunden bleiben kann. Da die bei dieser bekannten Brustprothese verwendeten Haftklebstoffe nach Lösen der Klebverbindung nicht unbegrenzt wiederverwendbar sind, sind die Haftbefestiger an dieser Brustprothese zweckmäßigerweise auswechselbar ausgeführt. Nachteilig und für die empfindliche, vernarbte Haut eine starke Beanspruchung ist jedoch bei dieser bekannten Brustprothese, daß nach dem Ankleben eine Sitzkorrektur nur dann vorgenommen werden kann, wenn die Brustprothese gelöst, in die richtige Sitzposition gebracht worden ist und dann erneut wieder angeheftet wird. Die Lebensdauer der Haftklebeeigenschaften der verwendeten Klebstoffe wird dadurch wesentlich herabgesetzt. Außerdem ist durch diese kleinflächigen und am Rand der Prothese angebrachten Befestigungsstellen kein sicherer Sitz der Prothese bei Bewegungen der Trägerin sichergestellt, da die Prothese in ihrem schwersten Bereich, d. h. in der Mitte, nur lose und unbefestigt auf der Haut aufliegt.

Demgegenüber ist es Aufgabe der vorliegenden Erfindung, eine Brustprothese mit einer körperauflageseitigen Haftfläche zu schaffen, die einen festen Sitz gewährleistet und auch nach dem Anheften an der Haut des menschlichen Körpers eine Drehkorrektur ermöglicht, so daß die richtige Sitzposition der Brustprothese ohne Wiederabnahme noch nachträglich eingestellt werden kann.

Eine weitere Aufgabe der vorliegenden Erfindung ist es, eine Brustprothese mit einer körperauflageseitigen Haftfläche zu schaffen, deren oberer Formteil trotz Beibehaltung der Brustform bei Berührungskontakt keine Unterschiede zur natürlichen Brust spürbar werden läßt und bei der die bei horizontalen und vertikalen Bewegungen des oberen Formteils auftretenden Bewegungsausschläge nicht direkt auf die Haftung der Brustprothese auf den Körper übertragen werden.

Zur Lösung dieser Aufgabe wird eine Brustprothese gemäß der eingangs beschriebenen Art vorgeschlagen, die erfindungsgemäß in der Weise ausgebildet ist, daß die Ausnehmung etwa mittig angeordnet und in Form eines rotationssymmetrischen Loches ausgebildet ist und der Brustformteil um die senkrechte Mittelachse des adapterartigen Formteils gegenüber diesem Formteil verdrehbar ist.

Durch die Ausgestaltung einer Brustprothese mit einem in ihrem Formteil drehbar gelagerten Aufklebeadapter, d. h., einem Brustformteil, der um die senkrechte Mittelachse des adapterartigen Formteils gegenüber diesem Formteil verdrehbar ist, ist der Trägerin einer derartigen Brustprothese die Möglichkeit gegeben, nach Anheften der Brustprothese an der Haut des Körpers eine Drehkorrektur der Brustprothese vorzunehmen, um die Brustprothese in die richtige Sitzposition zu bringen und somit u. a. auch eine Anpassung an das jeweils verwendete Büstenhalterfabrikat vornehmen zu können. Dadurch, daß nur der im Brustformteil drehbar gelagerte, adapterartige Formteil außenseitig den Haftklebstoff trägt, haftet die Brustprothese an der Körperhaut nicht im Bereich ihrer gesamten Auflagefläche an, sondern nur im Bereich des drehbaren adapterartigen Formteils, wodurch die Möglichkeit gegeben ist, eine Drehkorrektur an der Brustprothese jederzeit vornehmen zu können.

Weitere vorteilhafte Ausgestaltungen der Erfindung gehen aus den abhängigen Ansprüchen hervor.

In der Zeichnung ist der Gegenstand der Erfindung beispielsweise dargestellt, und zwar zeigt

Figur 1 eine Brustprothese mit einem im Brustformteil drehbar gehaltenen, adapterförmigen Formteil mit einer außenseitigen Beschichtung aus einer Haftklebemasse in einem senkrechten Schnitt und

Figur 2 der adapterartigen Formteil in einem senkrechten Schnitt.

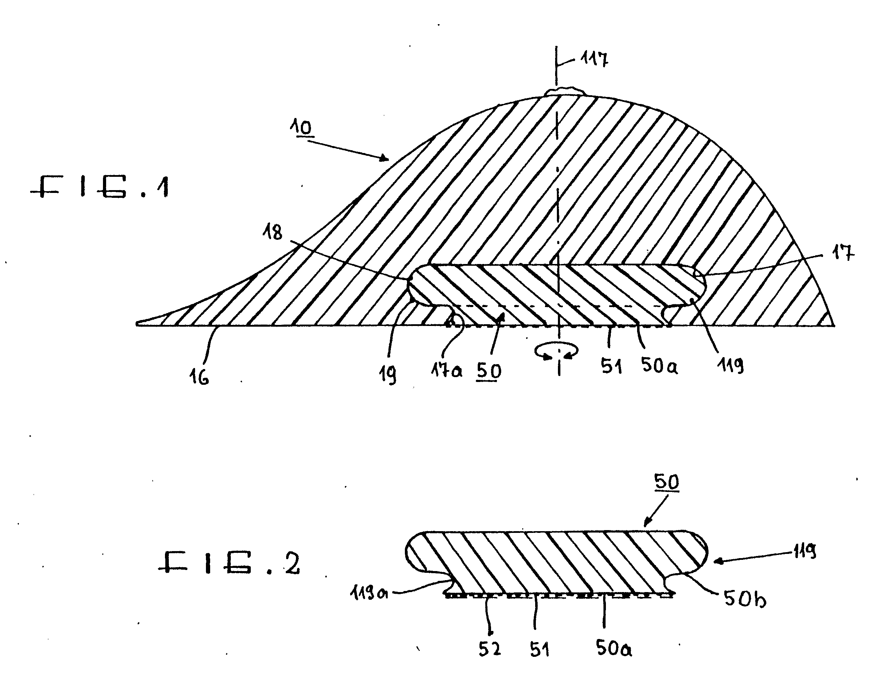

Bei der in Fig. 1 gezeigten Ausführungsform einer Brustprothese ist dessen Brustformteil mit 10 bezeichnet, der eine der weiblichen Brust entsprechende Form aufweist und als Vollkörper oder als Hohlkörper ausgebildet sein kann. Der Brustformteil 10 besteht aus einem elastischen, homogenen Kunststoff, insbesondere aus einem toxikologisch unbedenklichen, additionsvernetzten Silikonkautschuk. Insbesondere können auch durch Vernetzungssteuerung erhaltene weiche Silikone verwendet werden, die nicht, wie bekannt, durch Ölzusätze in ihrer Flexibilität eingestellt sind. Die Auflagefläche des Brustformteils ist bei 16 angedeutet.

Der Brustformteil 10 weist im Bereich seiner Auflagefläche 16, und zwar etwa mittig zum Formteil selbst eine Ausnehmung 17 auf, die zur Aufnahme und Halterung eines um seine senkrechte Mittelachse 117 verdrehbaren adapterartigen Formteils 50 ausgebildet ist, dessen außenseitige, in der Ebene der Auflagefläche 16 des Brustformteils liegende Wandfläche 50a mit einer hautverträglichen Beschichtung 51 aus einer Haftklebemasse versehen ist.

Die Ausnehmung 17 ist in Form eines rotationssymmetrischen Loches ausgebildet und weist einen kreisförmigen Querschnitt auf. Der adapterartige Formteil 50 ist kreisscheibenförmig ausgebildet und besteht vorteilhafterweise aus dem gleichen Material, aus dem auch der Brustformteil 10 der Brustprothese hergestellt ist. Es besteht jedoch auch die Möglichkeit, für die Herstellung des adapterartigen Formteils 50 einen anderen Werkstoff zu verwenden, jedoch ist es wesentlich, daß Werkstoffe mit einer gewissen Flexibilität und Anpassungsfähigkeit an den Körper verwendet werden.

Als Haftklebemasse für die Beschichtung 51 an der Außenwandfläche 50a des adapterartigen Formteils 50 können alle diejenigen Klebemassen Verwendung finden, die toxikologisch unbedenklich sind. Besteht der Brustformteil 10 aus einem additionsvernetzten Silikonkautschuk, dann ist die dem Körper der Trägerin der Brustprothese zugewandte Klebefläche des Formteils 50 vorzugsweise aus der gleichen Kautschukmasse als Klebemasse abgeleitet. Klebemasse und der adapterartige Formteil 50 sind bei der Vulkanisation zusammengewachsen.

Zur Gewährleistung einer Drehbarkeit des adapterartigen Formteils 50 in dem Brustformteil 10 ist die in diesem ausgebildete Ausnehmung 17 an ihrer Innenwandfläche 17a mit einem ringförmigen Führungsprofil 19 versehen, in das ein an der Umlaufwandung 50b des scheibenförmigen Formteils 50 angeformtes Gegenprofil 119 eingreift und in dem Führungsprofil 19 gehalten ist.

Bei dem in Fig. 1 gezeigten Ausführungsbeispiel ist die Ausnehmung 17 von einem ringförmigen Wandabschnitt 17a gebildet, der in die Auflagefläche 16 des Brustformteils 10 übergeht und an den sich ein ringnutartiger Abschnitt 18 anschließt. Der adapterartige Formteil 50 weist an seiner Umfangswandung 50b ein in das von dem ringförmigen Wandabschnitt 17a und dem ringnutartigen Abschnitt 18 gebildete Führungsprofil 19 eingreifendes Profil 119a als Gegenprofil 119 auf, so daß der adapterartige Formteil 50 aufgrund seiner Ausgestaltung sprengringartig in der Ausnehmung 17 gehalten ist. Ein derart ausgebildetes adapterartiges Formteil 50 ist mühelos in die Ausnehmung 17 einsetzbar. Aufgrund der kreisscheibenförmigen Ausbildung des Formteils 50 ist nach dem Anheften der Brustprothese an dere Körperhaut der die Brustprothese tragenden Person eine Drehkorrektur der Brustprothese möglich.

Bei einem Nachlassen der Klebewirkung des Formteils 50 ist ein neues klebkraftaufweisendes Formteil 50 mühelos in die Ausnehmung 17 der vorhandenen Brustprothese einsetzbar.

Ist der Brustformteil 10 als Hohlkörper ausgebildet, dann weist der Brustformteil im Bereich seiner Auflagefläche 16 eine halsartige, sich in den Innenraum des hohlkörperartigen Brustformteils erstreckende Einziehung auf, die zur Erhaltung einer gewissen Formstabilität des Brustformteils mit Stützrippen versehen sein kann. Im Innenwandbereich weist dann diese halsartige Einziehung das Führungsprofil 19 auf, in das das Gegenprofil 119 des adapterartigen Formteils 50 einsetzbar ist, so daß die freie Drehbeweglichkeit der Brustprothese gewährleistet ist.

Die Beschichtung 51 auf der Außenwandfläche 50a des Formteils 50 ist mittels eines abziehbaren Schutzblattes 52 abgedeckt, welches vor dem Anheften der Brustprothese an die Körperhaut abgezogen wird (Fig. 2).

In einer bevorzugten Ausführungsform der Erfindung weist der Brustformteil 10 in seinem oberen Teil, insbesondere seinem Warzenbereich, einen gegenüber dem Brustformteilmaterial hochflexibel ausgebildeten Weichteilkern auf.

Eine erfindungsgemäße Ausführungsform besteht dabei darin, daß zur Ausbildung dieses Weichteilkerns der Brustformteil in seinem oberen Teil, d. h. insbesondere in seinem Warzenbereich, einen als dünne Haut ausgebildeten oberen Wandabschnitt und in seinem unteren Teil ebenfalls einen als dünne Haut ausgebildeten unteren Wandabschnitt aufweist, wobei beide Wandabschnitte unter Ausbildung eines blasenartigen, geschlossenen Hohlraums miteinander verbunden sind, der mit einem Silikon-Gel-Körper ausgefüllt ist.

Aufgrund dieser erfindungsgemäßen Ausgestaltung ist eine Brustprothese mit einem weichen oberen Formteil geschaffen, so daß bei Berührungskontakt keine Unterschiede zur natürlichen Brust spürbar werden. Dadurch, daß der vordere, gallertartige Teil der Brustprothese horizontale und vertikale Bewegungen ausführen kann, wird eine direkte Übertragung dieser Bewegungsausschläge auf die Körperhaftung vermieden.

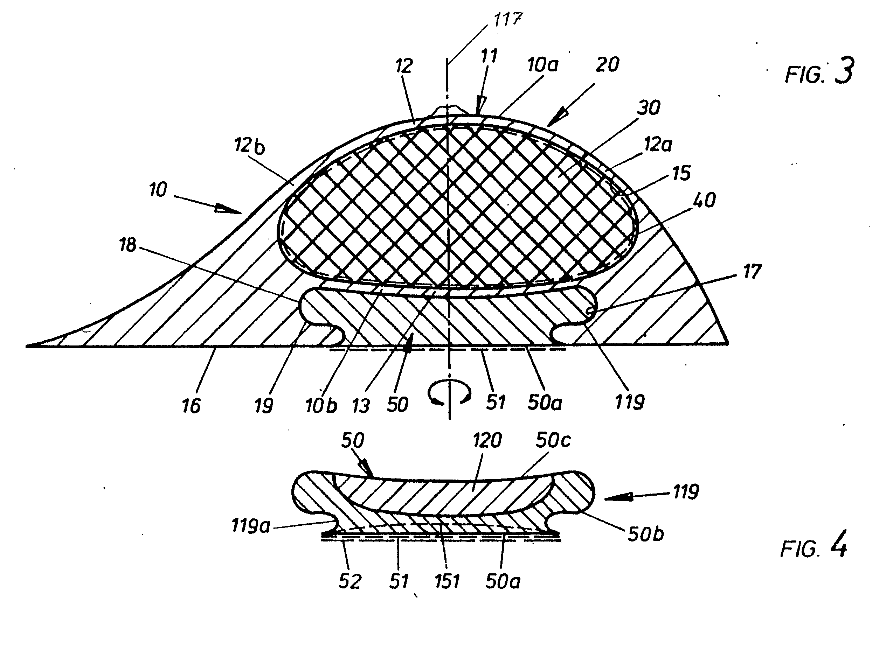

In den Figuren 3 und 4 ist diese Ausführungsform der Erfindung beispielsweise dargestellt, und zwar zeigt in senkrechten Schnitten

Figur 3 eine Brustprothese mit im oberen Teil ihres Brustformteils angeordnetem Weichteilkern und mit einem in ihrem Brustformteil drehbar gehaltenen, adapterförmigen Formteil mit einer außenseitigen Beschichtung aus einer Haftklebemasse und

Figur 4 den adapterartigen Formteil.

Die Brustprothese besteht gemäß Fig. 3 aus einem Brustformteil 10 aus einem elastischen, homogenen Kunststoff, insbesondere aus einem toxikologisch unbedenklichen, additionsvernetzten Silikonkautschuk. Der obere mit 11 bezeichnete Teil des Brustformteils 10 ist entsprechend der weiblichen Brust mit Warze und Warzenhof ausgestattet. Die Auflagefläche des Brustformteils 10 ist bei 16 angedeutet. Insbesondere werden durch Vernetzungssteuerung erhaltene weiche Silikone verwendet, die nicht, wie bekannt, durch Ölzusätze in ihrer Flexibilität eingestellt sind.

Der Brustformteil 10 weist im Bereich seines oberen Teiles 11 einen gegenüber dem Brustformteilmaterial hochflexibel ausgebildeten Weichteilkern 20 auf, der im oberen vorderen Teil 10a des Brustformteils 10 ausgebildet ist und sich bis in den unteren Teil 10b des Brustformteils 10 erstreckt.

Zur Ausbildung dieses Weichteilkerns 20 weist der Brustformteil 10 in seinem oberen vorderen Teil 10a einen Wandabschnitt 12 auf, der als dünne Haut ausgebildet ist. Dieser hautartige Wandabschnitt 12 ist bis zum Mittelteil des Brustformteils hin fortgezogen, so daß ein dem Brustformteil entsprechender blasenartiger, geschlossener Hohlraum 15 ausgebildet ist, der mit einem untervernetzten Silikon-Gel-Körper 30 ausgefüllt ist. Die sich an den hautartig dünnen Wandabschnitt 12 anschließenden seitlichen Wandungen 12a und 12b sind zum oberen Teil 11 des Brustformteils 10 hin gleichmäßig konisch abnehmend ausgebildet, da sonst bei der Eindellung eine Kantenbildung in der äußeren Form des Brustformteils 10 entstehen würde. Der unter dem Wandabschnitt 12 liegende und im unteren Teil 10b den Hohlraum 15 begrenzende Wandabschnitt 13 ist ebenfalls als dünne Haut entsprechend dem Wandabschnitt 12 ausgebildet.

Der hochflexible Weichteilkörper 20 des Brustformteils 10 bildet eine einwandfreie, dem Körper angepaßte Anlagefläche, da er die Druckkräfte, die durch einen Büstenhalter auf die Brustprothese übertragen werden, nicht formstabil aufnehmen kann. Hierfür wird insbesondere der untere, tragende Brustprothesenanteil aus einem höher vernetzten Material gefertigt.

Der Silikon-Gel-Körper 30 ist nach Einbringen in den Hohlraum 15 des Brustformteils 10 mit den

den Hohlraum begrenzenden Wandflächen 12, 12a, 12b, 13 während des Vulkanisationsprozesses zusammengewachsen. Das Einbringen des Silikon-Gels in den Hohlraum 15 kann z. B. vermittels einer nach Beendigung des Einfüllvorganges verschließbaren Öffnung in dem Wandabschnitt 12 erfolgen.

Um zu verhindern, daß die Masse der Silikon-Gel-Körpers 30 durch die dünnen Wandabschnitte 12 und 13 hindurchwandern kann bzw. um zu vermeiden, daß Weichmacheranteile des Silikon-Gel-Körpers durch diese Wandabschnitte hindurchdiffundieren können, ist die den Hohlraum 15 begrenzende Wandfläche ausgekleidet mit einer Kunststoffolie, die weichmacherundurchlässig ist und die ein Hindurchdiffundieren der klebrigen Silikon-Gel-Masse des Körpers 30 verhindert. Vorteilhafterweise wird als Auskleidungsfolie 40 eine Polyurethanfolie verwendet.

Wie Fig. 4 zeigt, kann der adapterartige Formteil 50 im Bereich seiner oberen Wandfläche 50c einen Weichteilkern 120 aufweisen, der gegenüber dem Material, aus dem der Formteil 50 hergestellt ist, hochflexibel ist. Durch diesen Weichteilkern 120 wird eine bessere Anschmiegsamkeit der Brustprothese an den von der Trägerin einer derartig ausgebildeten Brustprothese getragenen Büstenhalter erreicht, denn aufgrund dieser speziellen Ausgestaltung des Formteils 50 nimmt dieser an den Gesamteigenschaften des Brustformteils 10 teil ohne auf diesen formstabilisierend einzuwirken. Hinzu kommt noch, daß die mit dem Weichteilkern 20 in dem Brustformteil 10 erzielte Wirkung, nämlich bei Berührungskontakt keine Unterschiede zur natürlichen Brust spürbar werden zu lassen, in keiner Weise beeinträchtigt wird, da Druckeinwirkungen auf den oberen Teil des Brustformteils 20 durch die hautartigen unteren Wandabschnitte 13 auf den Weichteilkern 120 in dem adapterartigen Formteil 50 übertragen werden und von diesem Druckkräfte aufgenommen werden, was zur Folge hat, daß die Druckkräfte über die Auflagefläche 16 des Brustformteils und auf die Körperhaut bei angelegter Brustprothese übertragen werden. Die Körperhaut wird somit im Auflagebereich entlastet.

Die Haftklebenmasse kann auf die Außenwandfläche 50a des adapterartigen Formteils 50 aufgetragen sein ; es besteht jedoch die Möglichkeit, die Haftklebenmasse in dem Formteil 50 zu integrieren, wie dies in Fig. 4 bei 151 angedeutet ist.

Um die erfindungsgemäßen Brustprothesen in ihrem Auflagebereich zusätzlich zu verfestigen und gegen ein Einreißen zu schützen, kann in die Auflagefläche 16 eine Textileinlage, beispielsweise aus hochreißfesten Polyamid-Fäden, eingelassen sein. Vorzugsweise ist die Textileinlage anlagenflächenseitig von einer Kunststoffschicht abgedeckt.

Die Brustprothesen können bei Bedarf, d. h. wenn es für die Trägerin beispielsweise zeitweilig angenehmer ist, keine Verklebung auf der Haut vorzunehmen, auch ohne das adapterartige Formteil als Einlage im Büstenhalter getragen werden. Hierbei wirkt sich der ringnutartige Abschnitt 18 insofern vorteilhaft aus, als er ein möglichst naturgetreues Schwingen der Brustprothese, insbesondere des Gel-Körpers 30, ermöglicht.

**Patentansprüche**

1. Brustprothese aus einem elastischen Kunststoff, insbesondere aus Silikonkautschuk-Masse, mit einem der weiblichen Brust entsprechenden Brustformteil (10) mit einer der menschlichen Körperform angepaßten Anlagefläche (16) wobei der Brustformteil (10) körperauflageflächenseitig eine Ausnehmung (17) und einen in dieser gehaltener adapterartigen Formteil (50) aufweist, dessen außenseitige, dem Körper zugewandte Wandfläche (50a) mit einer Beschichtung (51) aus einer hautverträglichen Haftklebemasse versehen ist, dadurch gekennzeichnet, daß die Ausnehmung (17) etwa mittig angeordnet und in Form eines rotationssymmetrischen Loches ausgebildet ist und der Brustformteil (10) um die senkrechte Mittelachse (117) des adapterartigen Formteils (50) gegenüber diesem Formteil (50) verdrehbar ist.

2. Brustprothese nach Anspruch 1, dadurch gekennzeichnet, daß die Ausnehmung (17) in dem Brustformteil (10) an ihrer Innenwandfläche (17a) ein ringförmiges Führungsprofil (19) aufweist, in dem ein an der Umlaufwandung (50b) des kreisscheibenförmigen Formteils (50) angeformtes Gegenprofil (119) eingreifend gehalten ist.

3. Brustprothese nach Anspruch 1 und 2, dadurch gekennzeichnet, daß die Seitenwandung der in Form eines rotationssymmetrischen Loches ausgebildeten Ausnehmung (17) von einem ringförmigen, in die Anlagefläche (16) des Brustformteils (10) mündenden Wandabschnitt (17a) gebildet ist, an den sich ein ringnutartiger Abschnitt (18) anschließt, während der Formteil (50) an seiner Umfangswandung (50b) ein in das vom ringförmigen Wandabschnitt (17a) und dem ringnutartigen Abschnitt (18) gebildeten Führungsprofil (19) eingreifendes Profil (119a) aufweist.

4. Brustprothese nach Anspruch 1 bis 3, dadurch gekennzeichnet, daß die Beschichtung (51) aus der Haftklebemasse mittels eines abziehbaren Schutzblattes (52) abgedeckt ist.

5. Brustprothese nach Anspruch 1 bis 4, dadurch gekennzeichnet, daß der Brustformteil (10) in seinem oberen Teil, insbesondere in seinem Warzenbereich (11), einen gegenüber dem Brustformteilmaterial hochflexibel ausgebildeten Weichteilkern (20) aufweist.

6. Brustprothese nach Anspruch 5, dadurch gekennzeichnet, daß der hochflexible Weichteilkern (20) im oberen vorderen Teil (10a) des Brustformteils (10) ausgebildet ist.

7. Brustprothese nach Anspruch 5 und 6, dadurch gekennzeichnet, daß der hochflexible Weichteilkern (20) sich bis in den unteren Teil (10b) des Brustformteils (10) erstreckt.

8. Brustprothese nach Anspruch 5 bis 7, dadurch gekennzeichnet, daß zur Ausbildung des Weichteilkerns (20) der Brustformteil (10) in seinem oberen Teil (11), insbesondere in seinem Warzenbereich, einen als dünne Haut ausgebildeten oberen Wandabschnitt (12) und in seinem unteren Teil (10b) einen als dünne Haut ausgebildeten unteren Wandabschnitt (13) aufweist, wobei beide Wandabschnitte (12, 13) unter Ausbildung eines blasenartigen, geschlossenen Hohlraumes (15) miteinander verbunden sind, der mit einem Silikon-Gel-Körper (30) ausgefüllt ist.

9. Brustprothese nach Anspruch 5 bis 8, dadurch gekennzeichnet, daß die den Silikon-Gel-Körper (30) seitlich begrenzenden Wandungen (12a, 12b) des Brustformteils (10) zu seinem oberen Teil (11) hin sich gleichmäßig konisch abnehmend erstreckend ausgebildet sind.

10. Brustprothese nach Anspruch 5 bis 9, dadurch gekennzeichnet, daß der Silikon-Gel-Körper (30) und die hautartigen Wandungen (12, 12a, 12b, 13) des Brustformteils (10) beim Vulkanisationsprozeß in ihren Anlagebereichen miteinander integrierend ausgebildet sind.

11. Brustprothese nach Anspruch 5 bis 10, dadurch gekennzeichnet, daß die den Hohlraum (15) begrenzende Wandfläche (12, 12a, 12b, 13) des Brustformteils (10) innenwandseitig mit einer das Durchdringen der Silikon-Gel-Körper-Komponenten durch den Brustformteil (10) verhindernden Folie (40) ausgekleidet ist.

12. Brustprothese nach Anspruch 1 bis 11, dadurch gekennzeichnet, daß der adapterförmige Formteil (50) im Bereich seiner oberen Wandfläche (50c) einen gegenüber dem Formteilmaterial hochflexibel ausgebildeten Weichteilkern (120) aufweist.

**Claims**

1. Mammary prosthesis of a resilient plastic, in particular of a silicone rubber composition, with a breast moulding (10) corresponding to the female breast and having a contact surface (16) adapted to the shape of the human body, the breast moulding (10) having a recess (17) on the side of the body-contact surface and an adaptor-like moulding (50) which is held in this recess and of which the outside wall surface (50a) facing the body is provided with a coating (51) of a pressure-sensitive adhesive tolerated by the skin, characterised in that the recess (17) is located approximately in the centre and is formed in the shape of a rotationally symmetrical hole, and the breast moulding (10) is rotatable relative to the adaptor-like moulding (50) about the vertical centre axis (117) of this moulding (50).

2. Mammary prosthesis according to Claim 1, characterised in that the recess (17) in the breast moulding (10) has, on its inner wall surface (17a), an annular guide profile (19) in which a counter-profile (119), which is moulded to the circumferential wall (50b) of the moulding (50) in the shape of a circular disc, is held in engagement.

3. Mammary prosthesis according to Claim 1 and 2, characterised in that the side wall of the recess (17), shaped in the form of a rotationally symmetrical hole is formed by an annular wall section (17a) which merges into the contact surface (16) of the breast moulding (10) and which is adjoined by a section (18) in the form of an annular groove, whilst the moulding (50) exhibits, on its circumferential wall (50b), a profile (119a) which engages in the guide profile (19) formed by the annular wall section (17a) and the section (18) in the form of an annular groove.

4. Mammary prosthesis according to Claims 1 to 3, characterised in that the coating (51) of a pressure-sensitive adhesive is covered by means of a peel-off backing sheet (52).

5. Mammary prosthesis according to Claims 1 to 4, characterised in that the breast moulding (10) has, in its upper part, in particular in its nipple region (11), a soft part core (20) which is made highly flexible as compared with the material of the breast moulding.

6. Mammary prosthesis according to Claim 5, characterised in that the highly flexible soft part core (20) is formed in the upper front part (10a) of the breast moulding (10).

7. Mammary prosthesis according to Claims 5 and 6, characterised in that the highly flexible soft part core (20) extends as far as the lower part (10b) of the breast moulding (10).

8. Mammary prosthesis according to Claims 5 to 7, characterised in that, in order to form the soft part core (20), the breast moulding (10) has, in its upper part (11), in particular in its nipple region, an upper wall section (12) in the shape of a thin skin and, in its lower part (10b), a lower wall section (13) in the shape of a thin skin, the two wall sections (12, 13) being mutually joined with the formation of a bag-like closed cavity (15) which is filled with a silicone gel body (30).

9. Mammary prosthesis according to Claims 5 to 8, characterised in that the walls (12a, 12b), laterally bounding the silicone gel body (30) of the breast moulding (10) have a shape extending with a uniform conical taper towards its upper part (11).

10. Mammary prosthesis according to Claims 5 to 9, characterised in that the silicone gel body (30) and the skin-like walls (12, 12a, 12b, 13) of the breast moulding (10) are formed mutually integrally in their contact regions during the vulcanisation process.

11. Mammary prosthesis according to Claims 5 to 10, characterised in that the wall surface (12, 12a, 12b, 13), bounding the cavity (15) of the breast moulding (10) is lined on the inner wall side with a sheet (40) which prevents penetration of the silicone gel body components through the breast moulding (10).

12. Mammary prosthesis according to Claims 1 to 11, characterised in that the adaptor-like moulding (50) has, in the region of its upper wall surface (50c), a soft part core (120) which is made highly flexible as compared with the moulding material.

## Revendications

1. Prothèse mammaire en matière plastique élastique, faite en particulier d'une masse en caoutchouc de silicone, qui comporte un élément façonné en forme de sein (10) à l'image du sein de la femme présentant une surface d'application (16) adaptée à la forme du corps humain, l'élément façonné en forme de sein (10) présentant du côté appliqué sur le corps, un évidement (17) et, maintenu dans celui-ci, un élément façonné de fixation (50) dont la face extérieure (50a) tournée vers le corps est munie d'un revêtement (51) d'une masse adhésive compatible avec la peau, caractérisée en ce que l'évidement (17) est ménagé à peu près centralement et réalisé sous la forme d'un trou à symétrie de rotation et l'élément façonné en forme de sein (10) peut tourner autour de l'axe vertical central (117) de l'élément façonné de fixation (50) par rapport à cet élément façonné (50).

2. Prothèse mammaire suivant la revendication 1, caractérisée en ce que l'évidement (17) dans l'élément façonné en forme de sein (10) présente à sa paroi intérieure (17a) un profil de guidage annulaire (19) dans lequel est retenu un profil de contre-partie (119) formé sur la paroi extérieure (50b) de l'élément façonné (50) en forme de disque circulaire.

3. Prothèse mammaire suivant les revendications 1 et 2, caractérisée en ce que la paroi latérale de l'évidement (17) réalisé en forme de trou à symétrie de rotation est formée par un segment de paroi annulaire (17a) débouchant dans la surface d'application (16) de l'élément façonné en forme de sein (10), auquel se raccorde un segment en gorge annulaire (18), tandis que l'élément façonné (50) présente à sa paroi périphérique (50b) un profil (119a) s'insérant dans le profil de guidage (19) formé par le segment de paroi annulaire (17a) et le segment en gorge annulaire (18).

4. Prothèse mammaire suivant les revendications 1 à 3, caractérisée en ce que le revêtement (51) de masse adhésive est recouvert d'une feuille de garde (52) à arracher.

5. Prothèse mammaire suivant les revendications 1 à 4, caractérisée en ce que l'élément façonné en forme de sein (10) présente dans sa partie supérieure, en particulier dans la région du mamelon (11), une âme formant partie molle (20) de nature très flexible en comparaison de la matière de l'élément façonné en forme de sein.

6. Prothèse mammaire suivant la revendication 5, caractérisée en ce que l'âme formant partie molle (20) très flexible est ménagée dans la partie supérieure antérieure (10a) de l'élément façonné en forme de sein (10).

7. Prothèse mammaire suivant les revendications 5 et 6, caractérisée en ce que l'âme formant partie molle (20) très flexible s'étend jusque dans la partie inférieure (10b) de l'élément façonné en forme de sein (10).

8. Prothèse mammaire suivant les revendications 5 à 7, caractérisée en ce que pour la réalisation de l'âme formant partie molle (20), l'élément façonné en forme de sein (10) présente dans sa partie supérieure (11), en particulier dans la région du mamelon, un segment de paroi supérieure (12) constitué par une peau mince et dans sa partie inférieure (10b), un segment de paroi inférieure (13) constitué par une peau mince, les deux segments de paroi (12, 13) étant réunis en formant un volume creux (15) vésiculaire fermé qui est rempli par un corps en gel de silicone (30).

9. Prothèse mammaire suivant les revendications 5 à 8, caractérisée en ce que les parois (12a, 12b) délimitant latéralement le corps en gel de silicone (30) de l'élément façonné en forme de sein (10) s'étendant en cône allant en diminuant régulièrement vers sa partie supérieure (11).

10. Prothèse mammaire suivant les revendications 5 à 9, caractérisée en ce que le corps en gel de silicone (30) et les parois de type peau (12, 12a, 12b, 13) de l'élément façonné en forme de sein (10) sont tels que lors du processus de vulcanisation, ils sont amenés à être d'une seule venue dans leur région de contact.

11. Prothèse mammaire suivant les revendications 5 à 10, caractérisée en ce que les surfaces de parois (12, 12a, 12b et 13) de l'élément façonné en forme de sein (10) délimitant le volume creux (15) sont revêtues du côté intérieur d'une pellicule (40) empêchant la pénétration des composants du corps en gel de silicone à travers l'élément en forme de sein (10).

12. Prothèse mammaire suivant les revendications 1 à 11, caractérisée en ce que l'élément façonné de fixation (50) présente dans la région de sa surface de paroi supérieure (50c) une âme formant partie molle (120) de nature très flexible en comparaison de la matière de l'élément façonné.

FIG.1

FIG.2

0 054 197

FIG. 3

FIG. 4

0 054 197